(19) Europäisches Patentamt
European Patent Office
Office européen des brevets

(11) EP 2 409 717 A2

(12) EUROPEAN PATENT APPLICATION

(43) Date of publication:
25.01.2012 Bulletin 2012/04

(51) Int Cl.:
*A61L 12/08* (2006.01)

(21) Application number: 11186067.2

(22) Date of filing: 01.11.2007

(84) Designated Contracting States:
AT BE BG CH CY CZ DE DK EE ES FI FR GB GR HU IE IS IT LI LT LU LV MC MT NL PL PT RO SE SI SK TR

(30) Priority: 20.12.2006 US 613356

(62) Document number(s) of the earlier application(s) in accordance with Art. 76 EPC:
07863744.4 / 2 094 321

(71) Applicant: Bausch & Lomb Incorporated
Rochester, NY 14604-2701 (US)

(72) Inventors:
- Xia, Erning
Penfield, NY New York 14526 (US)
- Hook, Daniel J.
Fairport, NY New York 14450 (US)
- Salamone, Joseph C.
San Antonio, TX Texas 78230 (US)

(74) Representative: Glas, Holger
Maiwald Patentanwalts GmbH
Elisenhof
Elisenstrasse 3
80335 München (DE)

Remarks:
This application was filed on 21-10-2011 as a divisional application to the application mentioned under INID code 62.

# (54) Packaging solutions

(57) Packaging system for storing an ophthalmic device in an aqueous packaging solution comprising an anionic polymer and a non-ionic polyol. Such solutions can be retained on the surface of an unused lens for extended periods of time, resulting in surface modification that persists in the eye, which may provide significant improvement in the wetting properties of fresh contact lenses used for the first time and, moreover, even several hours after lens insertion, preventing dryness and improving lubricity.

EP 2 409 717 A2

## Description

### BACKGROUND OF THE INVENTION

1. Technical Field

The present invention generally relates to packaging solutions for ophthalmic devices such as contact lenses.

2. Description of Related Art

Blister-packs and glass vials are typically used to individually package each soft contact lens for sale to a customer. Saline or deionized water is commonly used to store the lens in the blister-packs, as mentioned in various patents related to the packaging or manufacturing of contact lenses. Because lens material may tend to stick to itself and to the lens package, packaging solutions for blister-packs have sometimes been formulated to reduce or eliminate lens folding and sticking. For this reason, poly(vinyl alcohol) (PVA) has been used in contact lens packaging solutions.

It has been stated that if a lens is thoroughly cleaned before insertion, lacrimal fluid can adequately wet the lens. Furthermore, the difficulties of adding a surfactant to a packaging solution, including the possibility of lowering shelf-life and/or adverse reactions during heat sterilization, have further limited the use of surfactants in a packaging solution for the purpose of providing any possible or marginal effect on lens comfort. It is only after a lens has been worn, when proteins or other deposits have formed on the surface of the lens, that surfactants have been used in standard lens-care solutions.

It is highly desirable that contact lens be as comfortable as possible for wearers. Manufacturers of contact lenses are continually working to improve the comfort of the lenses. Nevertheless, many people who wear contact lenses still experience dryness or eye irritation throughout the day and particularly towards the end of the day. An insufficiently wetted lens at any point in time will cause significant discomfort to the lens wearer. Although wetting drops can be used as needed to alleviate such discomfort, it would certainly be desirable if such discomfort did not arise in the first place.

U.S. Patent No. 5,882,687 discloses a package containing a contact lens suitable for immediate use which comprises (a) a solution comprising a soluble polyanionic component and having a viscosity of less than 50 cps at 25°C, an osmolality of at least about 200 mOsm/kg and a pH in the range of about 6 to about 9; (b) at least one contact lens, and (c) a container for holding the solution and contact lens sufficient to preserve the sterility of the solution and contact lens, wherein the solution contains no additional disinfectant component. However, a polyanion such as carboxymethylcellulose alone can possess a shape of a sphere or random coil and has a Mark-Houwink Constant ($\alpha$) of 0 when in the shape of a sphere and 0.5 to 0.8 when in the shape of a random coil. This type of polyanion may not coat uniformly a surface of a contact lens, resulting in the lens being relatively uncomfortable during use.

Accordingly, it would be desirable to provide an improved packaging system for ophthalmic devices such as an ophthalmic lens that the lens would be comfortable to wear in actual use and allow for extended wear of the lens without irritation or other adverse effects to the cornea.

### SUMMARY OF THE INVENTION

In accordance with one embodiment of the present invention, a method of preparing a package comprising a storable, sterile ophthalmic device is provided comprising:

(a) immersing an ophthalmic device in an aqueous packaging solution comprising an anionic polymer and a non-ionic polyol, wherein the aqueous packaging solution has an osmolality of at least about 200 mOsm/kg and a pH in the range of about 6 to about 9;
(b) packaging the solution and the device in a manner preventing contamination of the device by microorganisms; and
(c) sterilizing the packaged solution and device.

In accordance with a second embodiment of the present invention, a method for packaging and storing a contact lens is provided comprising, prior to delivery of the contact lens to the customer-wearer, immersing the contact lens in an aqueous packaging solution inside a package and heat sterilizing the solution, wherein the aqueous packaging solution comprises an anionic polymer and a non-ionic polyol, wherein the aqueous packaging solution has an osmolality of at least about 200 mOsm/kg and a pH of about 6 to about 9.

In accordance with a third embodiment of the present invention, a packaging system for the storage of an ophthalmic device is provided comprising a sealed container containing one or more unused ophthalmic devices immersed in an aqueous packaging solution comprising an anionic polymer and a non-ionic polyol, wherein the aqueous packaging solution has an osmolality of at least about 200 mOsm/kg, a pH of about 6 to about 9 and is heat sterilized.

In accordance with a fourth embodiment of the present invention, a packaging system for the storage of an ophthalmic device is provided comprising:

(a) an aqueous packaging solution comprising an anionic polymer and a non-ionic polyol, wherein the aqueous packaging solution has an osmolality of at least about 200 mOsm/kg and a pH in the range of about 6 to about 9;
(b) at least one ophthalmic device; and
(c) a container for holding the solution and ophthalmic device sufficient to preserve the sterility of the solution and ophthalmic device, wherein the solution does not contain an effective disinfecting amount of a disinfecting agent.

By combining a non-ionic polyol with an anionic polymer such as an anionic carboxymethylcellulose to form an aqueous packaging solution, a more uniform coating of the anionic polymer may be obtained. It is believed that the Mark-Houwink Constant ($\alpha$) of the anionic polymer increases to a level resulting in a coating that is more uniform over the lens, and hydrogen bonding of the anionic polymer is more effective. Thus, the lens will be more comfortable to wear in actual use and may allow for the extended wear of the lens without irritation or other adverse effects to the cornea.

## DETAILED DESCRIPTION OF THE PREFERRED EMBODIMENTS

The present invention provides a packaging system for the storage of ophthalmic devices intended for direct contact with body tissue or body fluid. As used herein, the term "ophthalmic device" refers to devices that reside in or on the eye. These lenses can provide optical correction, wound care, drug delivery, diagnostic functionality or cosmetic enhancement or effect or a combination of these properties. Representative examples of such devices include, but are not limited to, soft contact lenses, e.g., a soft, hydrogel lens; soft, non-hydrogel lens and the like, hard contact lenses, e.g., a hard, gas permeable lens material and the like, intraocular lenses, overlay lenses, ocular inserts, optical inserts and the like. As is understood by one skilled in the art, a lens is considered to be "soft" if it can be folded back upon itself without breaking. Any material known to produce an ophthalmic device including a contact lens can be used herein. It is particularly useful to employ biocompatible materials herein including both soft and rigid materials commonly used for ophthalmic lenses, including contact lenses. The preferred substrates are hydrogel materials, including silicone hydrogel materials and non-silicone hydrogel materials.

A wide variety of materials can be used herein. Hydrogels in general are a well-known class of materials that comprise hydrated, crosslinked polymeric systems containing water in an equilibrium state. Hydrogel contact lens materials are made from at least one hydrophilic monomer, such as 2-hydroxethyl methacrylate (HEMA), N-vinylpyrrolidone (NVP) or N,N-dimethylacrylamide (DMA). Hydrogels generally have a water content greater than about 15 weight percent and more commonly between about 20 to about 80 weight percent.

One class of hydrogels is silicone hydrogels. These materials are usually prepared by polymerizing a mixture containing at least one silicone-containing monomer and at least one hydrophilic monomer. Typically, either the silicone-containing monomer or the hydrophilic monomer functions as a crosslinking agent (a crosslinker being defined as a monomer having multiple polymerizable functionalities) or a separate crosslinker may be employed. Applicable silicone-containing monomeric units for use in the formation of silicone hydrogels are well known in the art and numerous examples are provided in U.S. Patent Nos. 4,136,250; 4,153,641; 4,740,533; 5,034,461; 5,070,215; 5,260,000; 5,310,779; and 5,358,995.

Representative examples of applicable silicon-containing monomeric units include bulky siloxanyl monomers represented by the structure of Formula I:

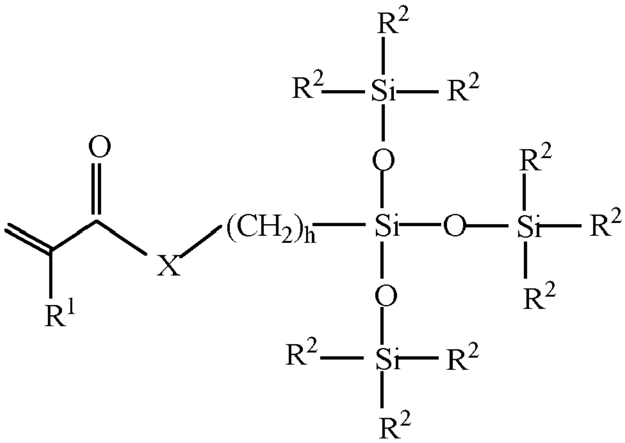

(I)

wherein X denotes -O- or -NR-; each $R^1$ independently denotes hydrogen or methyl; each $R^2$ independently denotes a lower alkyl radical, phenyl radical or a group represented by

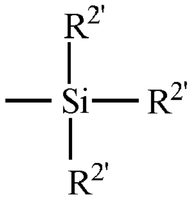

wherein each $R^{2'}$ independently denotes a lower alkyl or phenyl radical; and h is 1 to 10.

Examples of bulky monomers are 3-methacryloyloxypropyltris(trimethyl-siloxy)silane or tris(trimethylsiloxy)silylpropyl methacrylate, sometimes referred to as TRIS and tris(trimethylsiloxy)silylpropyl vinyl carbamate, sometimes referred to as TRIS-VC and the like.

Such bulky monomers may be copolymerized with a silicone macromonomer, such as a poly(organosiloxane) capped with an unsaturated group at two or more ends of the molecule. U.S. Patent No. 4,153,641 discloses, for example, various unsaturated groups such as acryloyloxy or methacryloyloxy groups.

Another class of representative silicone-containing monomers includes, but is not limited to, silicone-containing vinyl carbonate or vinyl carbamate monomers such as, for example, 1,3-bis[4-vinyloxycarbonyloxy)but-1-yl]tetramethyl-disiloxane; 3-(trimethylsilyl)propyl vinyl carbonate; 3-(vinyloxycarbonylthio)propyl-[tris(trimethylsiloxy)silane]; 3-[tris(trimethylsiloxy)silyl]propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; trimethylsilylmethyl vinyl carbonate and the like and mixtures thereof.

Another class of silicon-containing monomers includes polyurethane-polysiloxane macromonomers (also sometimes referred to as prepolymers), which may have hard-soft-hard blocks like traditional urethane elastomers. They may be end-capped with a hydrophilic monomer such as 2-hydroxyethyl methacrylate (HEMA). Examples of such silicone urethanes are disclosed in a variety or publications, including PCT Published Application No. WO 96/31792 discloses examples of such monomers, which disclosure is hereby incorporated by reference in its entirety. Representative examples of silicone urethane monomers are represented by Formulae II and III:

$$E(*D*A*D*G)_a *D*A*D*E'; \text{ or} \qquad (II)$$

or

$$E(*D*G*D*A)_a *D*A*D*E'; \text{ or} \qquad (III)$$

or

wherein:

D independently denotes an alkyl diradical, an alkyl cycloalkyl diradical, a cycloalkyl diradical, an aryl diradical or an alkylaryl diradical having 6 to about 30 carbon atoms;

G independently denotes an alkyl diradical, a cycloalkyl diradical, an alkyl cycloalkyl diradical, an aryl diradical or an alkylaryl diradical having 1 to about 40 carbon atoms and which may contain ether, thio or amine linkages in the main chain;

* denotes a urethane or ureido linkage;

a is at least 1;

A independently denotes a divalent polymeric radical of Formula IV:

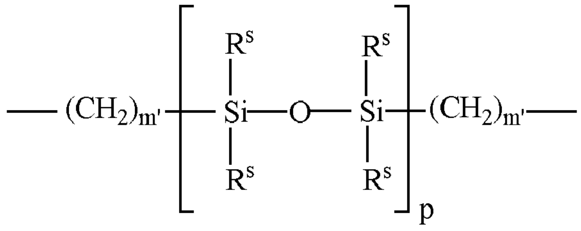

(IV)

wherein each $R^s$ independently denotes an alkyl or fluoro-substituted alkyl group having 1 to about 10 carbon atoms which may contain ether linkages between the carbon atoms; m' is at least 1; and p is a number that provides a moiety weight of about 400 to about 10,000;

each of E and E' independently denotes a polymerizable unsaturated organic radical represented by Formula V:

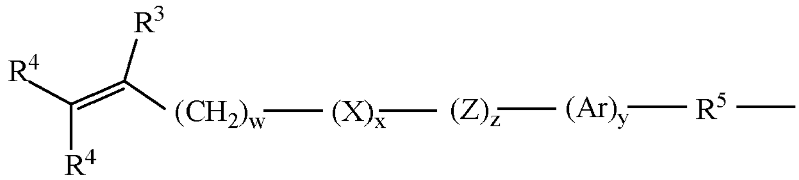

(V)

wherein: $R^3$ is hydrogen or methyl;

$R^4$ is hydrogen, an alkyl radical having 1 to 6 carbon atoms, or a -CO-Y-$R^6$ radical wherein Y is -O-, -S- or -NH-;

$R^5$ is a divalent alkylene radical having 1 to about 10 carbon atoms;

$R^6$ is a alkyl radical having 1 to about 12 carbon atoms;

X denotes -CO- or -OCO-;

Z denotes -O- or -NH-;

Ar denotes an aromatic radical having about 6 to about 30 carbon atoms;

w is 0 to 6; x is 0 or 1; y is 0 or 1; and z is 0 or 1.

A preferred silicone-containing urethane monomer is represented by Formula VI:

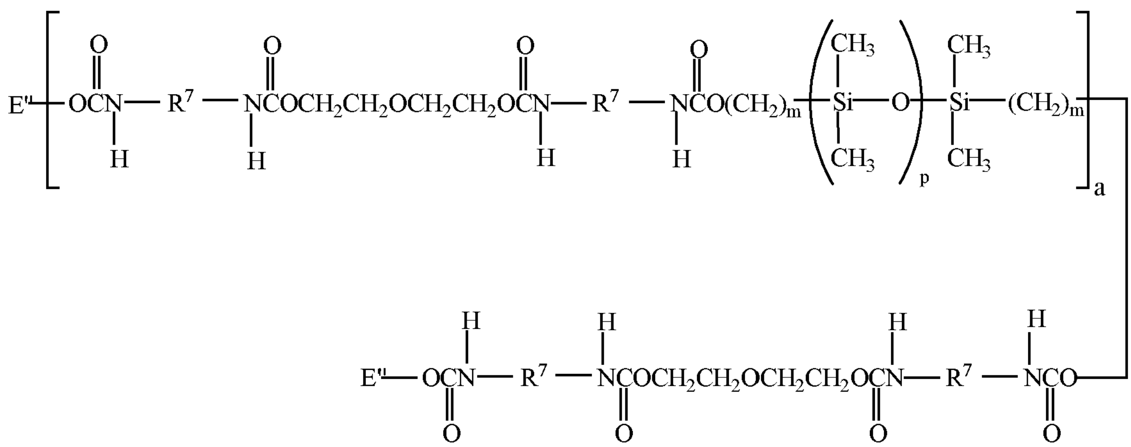

wherein m is at least 1 and is preferably 3 or 4, a is at least 1 and preferably is 1, p is a number which provides a moiety weight of about 400 to about 10,000 and is preferably at least about 30, $R^7$ is a diradical of a diisocyanate after removal of the isocyanate group, such as the diradical of isophorone diisocyanate, and each E" is a group represented by:

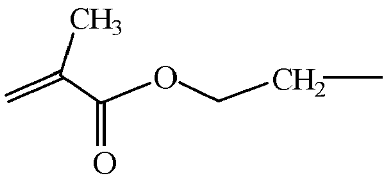

In another embodiment of the present invention, a silicone hydrogel material comprises (in bulk, that is, in the monomer mixture that is copolymerized) about 5 to about 50 percent, and preferably about 10 to about 25, by weight of one or more silicone macromonomers, about 5 to about 75 percent, and preferably about 30 to about 60 percent, by weight of one or more polysiloxanylalkyl (meth)acrylic monomers, and about 10 to about 50 percent, and preferably about 20 to about 40 percent, by weight of a hydrophilic monomer. In general, the silicone macromonomer is a poly (organosiloxane) capped with an unsaturated group at two or more ends of the molecule. In addition to the end groups in the above structural formulas, U.S. Patent No. 4,153,641 discloses additional unsaturated groups, including acryloyloxy or methacryloyloxy groups. Fumarate-containing materials such as those disclosed in U.S. Patent Nos. 5,310,779; 5,449,729 and 5,512,205 are also useful substrates in accordance with the invention. Preferably, the silane macromonomer is a silicon-containing vinyl carbonate or vinyl carbamate or a polyurethane-polysiloxane having one or more hard-soft-hard blocks and end-capped with a hydrophilic monomer.

Suitable hydrophilic monomers include amides such as N,N-dimethylacrylamide and N,N-dimethylmethacrylamide, cyclic lactams such as N-vinyl-2-pyrrolidone and poly(alkene glycols) functionalized with polymerizable groups. Examples of useful functionalized poly(alkene glycols) include poly(diethylene glycols) of varying chain length containing monomethacrylate or dimethacrylate end caps. In a preferred embodiment, the poly(alkene glycol) polymer contains at least two alkene glycol monomeric units. Still further examples are the hydrophilic vinyl carbonate or vinyl carbamate monomers disclosed in U.S. Patent No. 5,070,215, and the hydrophilic oxazolone monomers disclosed in U.S. Patent No. 4,910,277. Other suitable hydrophilic monomers will be apparent to one skilled in the art.

In one embodiment, the lens can be a Group II and Group IV lens having a water content greater than about 50% by weight, and preferably about 55% to about 80% water, although the invention is applicable for any type of soft hydrogel contact lens. Representative contact lens materials include, but are not limited to materials known by the following USAN and the USAP Dictionary of Drug Names: bufilcon A, etafilcon A, methafilcon A, ocufilcon C, perfilcon A, phemfilcon A, vifilcon A, hilafilcon A, hilafilcon B, balafilcon A, methafilcon B, ocufilcon D, methafilcon A, etafilcon A lidofilcon A or B, and alphafilcon A.

The above materials are merely exemplary, and other materials for use as substrates that can benefit by being packaged in the aqueous packaging solution according to the present invention and have been disclosed in various publications and are being continuously developed for use in ophthalmic devices such as contact lenses and other medical devices can also be used. For example, an ophthalmic device for use herein can be a cationic ophthalmic lens such as a cationic contact lens or the ophthalmic device can be fluorinated silicone-containing monomers. Such monomers have been used in the formation of fluorosilicone hydrogels to reduce the accumulation of deposits on contact lenses made therefrom, as disclosed in, for example, U.S. Patent Nos. 4,954,587; 5,010,141 and 5,079,319. The use of silicone-containing monomers having certain fluorinated side groups, i.e., $-(CF_2)-H$, have been found to improve compatibility between the hydrophilic and silicone-containing monomeric units. See, e.g., U.S. Patent Nos. 5,321,108 and 5,387,662.

In another embodiment, the present invention is also directed to a contact lens for extended-wear or specialty uses, such as for relatively thick lenses. Extended lenses are lenses capable of being worn overnight, preferably capable of being worn for at least one week, most preferably capable of wear for a continuous period of one week to one month. By "capable" is meant lenses approved by one or more governmental regulatory authorities for such consumer use, for example, the U.S. Food & Drug Administration (USFDA) in the U.S. or its equivalent in other countries.

Extended-wear lenses require relatively high oxygen permeability. The oxygen-permeability is the rate at which oxygen will pass through a material. The oxygen-permeability Dk of a lens material does not depend on lens thickness. Oxygen permeability is measured in terms of barrers which have the following units of measurement:

On the other hand, the oxygen transmissibility of a lens, as used herein, is the rate at which oxygen will pass through a specific lens. Oxygen transmissibility, Dk/t, is conventionally expressed in units of barrers/mm, where t is the average thickness of the material (in units of mm) over the area being measured. For example, a lens having a Dk of about 90 barrers (oxygen-permeability barrers) and a thickness of about 90 microns (about 0.090 mm) would have a Dk/t or about 100 barrers/mm (oxygen transmissibility barrers/mm).

Ophthalmic devices such as contact lenses for application of the present invention can be manufactured employing various conventional techniques, to yield a shaped article having the desired posterior and anterior lens surfaces. Spincasting methods are disclosed in U.S. Patent Nos. 3,408,429 and 3,660,545; preferred static casting methods are disclosed in U.S. Patent Nos. 4,113,224 and 4,197,266. Curing of the monomeric mixture is often followed by a machining operation in order to provide a contact lens having a desired final configuration. As an example, U.S. Patent No. 4,555,732 discloses a process in which an excess of a monomeric mixture is cured by spincasting in a mold to form a shaped article having an anterior lens surface and a relatively large thickness. The posterior surface of the cured spincast article is subsequently lathe cut to provide a contact lens having the desired thickness and posterior lens surface. Further machining operations may follow the lathe cutting of the lens surface, for example, edge-finishing operations.

After producing a lens having the desired final shape, it is desirable to remove residual solvent from the lens before edge-finishing operations. This is because, typically, an organic diluent is included in the initial monomeric mixture in order to minimize phase separation of polymerized products produced by polymerization of the monomeric mixture and to lower the glass transition temperature of the reacting polymeric mixture, which allows for a more efficient curing process and ultimately results in a more uniformly polymerized product. Sufficient uniformity of the initial monomeric mixture and the polymerized product are of particular concern for silicone hydrogels, primarily due to the inclusion of silicone-containing monomers which may tend to separate from the hydrophilic comonomer. Suitable organic diluents include, for example, monohydric alcohols such as $C_6$-$C_{10}$ straight-chained aliphatic monohydric alcohols, e.g., n-hexanol and n-nonanol; diols such as ethylene glycol; polyols such as glycerin; ethers such as diethylene glycol monoethyl ether; ketones such as methyl ethyl ketone; esters such as methyl enanthate; and hydrocarbons such as toluene. Preferably, the organic diluent is sufficiently volatile to facilitate its removal from a cured article by evaporation at or near ambient pressure. Generally, the diluent is included at about 5 to about 60 percent by weight of the monomeric mixture, with about 10 to about 50 percent by weight being especially preferred.

The cured lens is then subjected to solvent removal, which can be accomplished by evaporation at or near ambient pressure or under vacuum. An elevated temperature can be employed to shorten the time necessary to evaporate the diluent. The time, temperature and pressure conditions for the solvent removal step will vary depending on such factors as the volatility of the diluent and the specific monomeric components, as can be readily determined by one skilled in the art. According to a preferred embodiment, the temperature employed in the removal step is preferably at least about 50 °C, for example, about 60 °C to about 80 °C. A series of heating cycles in a linear oven under inert gas or vacuum may be used to optimize the efficiency of the solvent removal. The cured article after the diluent removal step should contain no more than twenty percent by weight of diluent, preferably no more than about 5 percent by weight or less.

Following removal of the organic diluent, the lens can then be subjected to mold release and optional machining operations. The machining step includes, for example, buffing or polishing a lens edge and/or surface. Generally, such machining processes may be performed before or after the article is released from a mold part. Preferably, the lens is dry released from the mold by employing vacuum tweezers to lift the lens from the mold, after which the lens is transferred by means of mechanical tweezers to a second set of vacuum tweezers and placed against a rotating surface to smooth the surface or edges. The lens may then be turned over in order to machine the other side of the lens

Next, the ophthalmic device will be immersed in an aqueous packaging solution containing at least an anionic polymer and a non-ionic polyol and stored in a packaging system according to the present invention. Generally, a packaging system for the storage of an ophthalmic device according to the present invention includes at least a sealed container containing one or more unused ophthalmic devices immersed in an aqueous packaging solution. Preferably, the sealed container is a hermetically sealed blister-pack, in which a concave well containing a contact lens is covered by a metal or plastic sheet adapted for peeling in order to open the blister-pack. The sealed container may be any suitable generally inert packaging material providing a reasonable degree of protection to the lens, preferably a plastic material such as polyalkylene, PVC, polyamide, and the like.

Any suitable anionic polymer may be employed in accordance with the present invention provided that it functions as described herein and has no substantial detrimental effect on the ophthalmic device such as a contact lens being stored or on the wearer of the lens. The anionic polymer is preferably ophthalmically acceptable at the concentrations used. The anionic polymer can include two (2) or more anionic (or negative) charges, preferably three (3) or more anionic (or negative) charges and most preferably ten (10) or more anionic (or negative) charges. As one skilled in the art will readily appreciate, the anionic polymer can include multiple charges in the same unit of the polymer or can include one or more charges on different repeating units in the polymer. Particularly useful anionic polymers are those which are water soluble, for example, soluble at the concentrations used in the presently useful liquid aqueous media, such as a liquid aqueous medium containing the anionic polymer. Particularly useful anionic polymers are those which are not eliminated during terminal sterilization of the packaged lenses.

In one embodiment, a class of anionic polymers includes one or more polymeric materials having multiple anionic charges. In one embodiment, an anionic polymer is an anionic polysaccharide. Representative examples of suitable anionic polymers for use herein include, but are not limited to, hyaluronic acid or a derivative thereof and/or

salts thereof; carboxymethylcelluloses; carboxymethylhydroxyethylcelluloses; carboxymethylstarch; carboxymethylhydroxyethylstarch; hydrolyzed polyacrylamides; hydrolyzed polyacrylonitriles; heparin; heparin sulfate, homopolymers and copolymers of one or more acrylic and methacrylic acids, acrylates and methacrylates; alginic acid or a derivative thereof and/or salts thereof; vinylsulfonic acid or a derivative thereof and/or salts thereof; polymers of amino acids such as polymers of aspartic acid, glutamic acid and the like or a derivative thereof and/or salts thereof; p-styrenesulfonic acid and the like or a derivative thereof and/or salts thereof; 2-methacryloyloxyethylsulfonic acids or a derivative thereof and/or salts thereof; 3-methacryloyloxy-2-hydroxypropylsulfonic acids or a derivative thereof and/or salts thereof; 2-acrylamido-2-methylpropanesulfonic acids or a derivative thereof and/or salts thereof; allylsulfonic acid or a derivative thereof and/or salts thereof; and the like and mixtures thereof. In one embodiment, an anionic polymer is an anionic polysaccharide. In another embodiment, an anionic polymer includes one or more of poly(acrylic acid), poly(methacrylic acid), polysaccharides, alginic acid, pectinic acid, carboxymethylcellulose, hyaluronic acid, heparin, heparin sulfate, carboxymethylchitosan, carboxymethylstarch, carboxymethyldextran, chondroitin sulfate, carboxymethylguar, any salts thereof, and mixtures thereof. The above list is intended for illustrative purposes only and not to limit the scope of the present invention. Such polymers are known to those of skill in the art.

In another embodiment, an anionic polymer includes one or more cellulose derivative, anionic polymers derived from acrylic acid (e.g., polymers derived from acrylic acid, acrylates and the like and mixtures thereof), anionic polymers derived from methacrylic acid (e.g., polymers derived from methacrylic acid, methacrylates, and the like and mixtures thereof), anionic polymers derived from alginic acid (e.g., polymers derived from alginic acid, alginates, and the like and mixtures thereof), anionic polymers derived from amino acids (e.g., polymers derived from amino acids, amino acid salts, and the like and mixtures thereof) and mixtures thereof. Particularly useful anionic polymers for use herein include cellulose polymers such as carboxymethylcelluloses.

The anionic polymer for use herein can have a Mark-Houwink Constant greater than 0.6, preferably greater than about 1 and more preferably greater than about 1.6. In one embodiment, the anionic polymer can have a Mark-Houwink Constant between about 1 to about 1.4. The Mark-Houwink constant ($\alpha$) is calculated using the technique disclosed in Introduction to Physical Polymer Science, Third Edition, L. H. Sperling, Wiley-Interscience, A John Wiley & Sons, Inc., Publication, New York, 2001. Interpretation of the Mark-Houwink constant for CMC is illustrated below in Table 1:

TABLE 1

| Values of the Mark-Houwink Constants ($\alpha$) | |
|---|---|
| Mark-Houwink Constants ($\alpha$) | Interpretation |
| 0 | Spheres |
| 0.5 -0.8 | Random coils |
| 1.0 | Stiff coils |
| 2.0 | Rods |

A Mark-Houwink Constant of zero is indicative of a spherical polymeric structure. A Mark-Houwink Constant between 0.5 and 0.8 indicates a physical configuration described as random coils. A Mark-Houwink Constant above 0.8 indicates a structure that is more ordered than random approaching a stiff coil. A Mark-Houwink Constant of about 1.0 is a stiff coil and a Mark-Houwink Constant of 2.0 represents a rod-like structure.

In one embodiment, the carboxy-containing polymer is an anionic carboxy-containing polysaccharide. Suitable polysaccharides of the present invention include carboxy-containing polysaccharides such as, for example, a carboxy-containing cellulose, hyaluronate, chondroitin sulfate, algin, pectin and xanthan. The anionic polymer could also be derived from carboxy-containing vinyl polymers, such as carbomers, poly(acrylic acid and poly(methacrylic acid), and derivatives thereof, or from anionic polypeptides, such as poly(glutamic acid) and poly(aspartic acid), and derivatives thereof.

In one embodiment, the average molecular weight of an anionic carboxy-containing polymer is a minimum of about 90 kDa and a maximum of about 700 kDa. Generally, the average molecular weight of the anionic carboxy-containing polymer is a minimum of about 150 kDa, preferably a minimum of about 200 kDa, and more preferably a minimum of about 250. The average molecular weight of the anionic carboxy-containing polymer is a maximum of about 650 kDa, preferably a maximum of about 600 kDa, more preferably a maximum of about 550 kDa and most preferably a maximum of about 500 kDa.

The amount of the anionic polymer present in the aqueous packaging solution is that amount effective to improve the surface properties of the ophthalmic device when combined with a non-ionic polyol. Preferably, the anionic

polymer is present in the packaging solution of the invention in an amount of at least about 0.01 % w/v. The specific amount of such anionic polymers used can vary widely depending on a number of factors, for example, the specific anionic polymer and non-ionic polyol being employed. Generally, the concentration of the anionic polymer is from about 0.01 to about 10 % w/w and preferably from about 0.5 to about 1.5 % w/w.

It is likewise preferable that the anionic polymer have a degree of substitution value that is a minimum of about 0.5 and a maximum of about 1.5. Preferably, the anionic polymer can have a degree of substitution value that is a minimum of about 0.9 to a maximum of about 1.1.

In one embodiment, the non-ionic polyol for use herein can be a non-ionic polyol containing 2 to about 12 carbon atoms and preferably 2 to 4 carbon atoms and from 2 to 6 hydroxyl groups. Representative examples of such non-ionic polyols include glycerin, ethylene glycol, poly(ethylene glycol), propylene glycol, sorbitol, mannitol, monosaccharides, disaccharides, and neutral oligo-polysaccharides, such as from methylcellulose, hydroxypropylmethylcellulose, guar, hydroxypropylguar, and oligomers of poly(vinyl alcohol) and derivatives thereof, and the like and mixtures thereof. In one embodiment, a non-ionic polyol can be glycerin, ethylene glycol, propylene glycol, sorbitol, mannitol, monosaccharides and mixtures thereof. In another embodiment, the non-ionic polyol can be disaccharides, oligosaccharides, poly(ethylene glycol) and mixtures thereof. In a preferred embodiment, the non-ionic polyol is glycerin.

The amount of non-ionic polyol present in the aqueous packaging solution will generally be an amount sufficient to increase the Mark-Houwink Constant of the anionic polymer in the solution such that a relatively more uniform coating can be formed on the surface of the device when packaged in a solution according to the present invention. In general, the concentration of the non-ionic polyol in the solution will ordinarily range from about 0.01 to about 10 % w/w and preferably from about 0.5 to about 3% w/w.

The aqueous packaging solutions according to the present invention are physiologically compatible. Specifically, the solution must be "ophthalmically safe" for use with a lens such as a contact lens, meaning that a contact lens treated with the solution is generally suitable and safe for direct placement on the eye without rinsing, that is, the solution is safe and comfortable for daily contact with the eye via a contact lens that has been wetted with the solution. An ophthalmically safe solution has a tonicity and pH that is compatible with the eye and includes materials, and amounts thereof, that are non-cytotoxic according to ISO standards and U.S. Food & Drug Administration (FDA) regulations. The solution should be sterile in that the absence of microbial contaminants in the product prior to release must be statistically demonstrated to the degree necessary for such products. The liquid media useful in the present invention are selected to have no substantial detrimental effect on the lens being treated or cared for and to allow or even facilitate the present lens treatment or treatments. The liquid media are preferably aqueous-based. A particularly useful aqueous liquid medium is that derived from saline, for example, a conventional saline solution or a conventional buffered saline solution.

The pH of the present aqueous packaging solutions should be maintained within the range of about 6.0 to about 9, and preferably about 6.5 to about 7.8. Suitable buffers may be added, such as boric acid, sodium borate, potassium citrate, citric acid, sodium bicarbonate, tris(hydroxymethyl)aminomethane, and various mixed phosphate buffers, e.g., combinations of $Na_2HPO_4$, $NaH_2PO_4$ and $KH_2PO_4$, and the like and mixtures thereof. Generally, buffers will be used in amounts ranging from about 0.05 to about 2.5 % by weight, and preferably from about 0.1 to about 1.5 % by weight of the solution.

Typically, the aqueous packaging solutions of the present invention are also adjusted with tonicity agents, to approximate the osmotic pressure of normal lacrimal fluids. The solutions are made substantially isotonic with physiological saline used alone or in combination, otherwise if simply blended with sterile water and made hypotonic or made hypertonic the lenses will lose their desirable optical parameters. Correspondingly, excess saline may result in the formation of a hypertonic solution which will cause stinging and eye irritation.

Examples of suitable tonicity adjusting agents include, but are not limited to, sodium and potassium chloride, dextrose, glycerin, calcium and magnesium chloride and the like and mixtures thereof. These agents are typically used individually in amounts ranging from about 0.01 to about 2.5 % w/v and preferably from about 0.2 to about 1.5 % w/v. Generally, a 0.9 % solution of sodium chloride is equivalent in osmolality to a 3 percent of glycerol solution or a 5 percent solution of monosaccharide, so the amount of a specific agent will vary depending on the agent used. Preferably, the tonicity agent will be employed in an amount to provide a final osmotic value of at least about 200 mOsm/kg, preferably from about 200 to about 400 mOsm/kg, and more preferably from about 250 to about 350 mOsm/kg.

If desired, one or more additional components can be included in the aqueous packaging solutions. Such additional component or components are chosen to impart or provide at least one beneficial or desired property to the packaging solution. Such additional components may be selected from components which are conventionally used in one or more ophthalmic device care compositions. Examples of such additional components include cleaning agents, wetting agents, nutrient agents, sequestering agents, viscosity builders, contact lens conditioning agents, antioxidants, and the like and mixtures thereof. These additional components may each be included in the packaging solutions in an amount effective to impart or provide the beneficial or desired property to the packaging solutions. For example, such additional components may be included in the packaging solutions in amounts similar to the amounts of such components used in other, e.g., conventional, contact lens care products.

Useful sequestering agents include, but are not limited to, disodium ethylenediaminetetraacetic acid (EDTA), alkali metal hexametaphosphate, citric acid, sodium citrate and the like and mixtures thereof.

Useful viscosity builders include, but are not limited to, hydroxyethylcellulose, methylcellulose, hydroxypropylmethylcellulose, poly(N-vinylpyrrolidone), guar, hydroxyethylguar, hydroxypropylguar, poly(vinyl alcohol) and the like and mixtures thereof.

Useful antioxidants include, but are not limited to, sodium metabisulfite, sodium thiosulfate, N-acetylcysteine, butylated hydroxyanisole, butylated hydroxytoluene and the like and mixtures thereof.

The method of packaging and storing an ophthalmic device such as a contact lens according to the present invention includes at least packaging an ophthalmic device immersed in the aqueous packaging solution described above. The method may include immersing the ophthalmic device in an aqueous solution prior to delivery to the customer/ wearer, directly following manufacture of the device. Alternately, the packaging and storing in the solution of the present invention may occur at an intermediate point before delivery to the ultimate customer (wearer) but following manufacture and transportation of the lens in a dry state, wherein the dry lens is hydrated by immersing the lens in the aqueous packaging solution. Consequently, a package for delivery to a customer may include a sealed container containing one or more unused contact lenses immersed in an aqueous packaging solution according to the present invention.

In one embodiment, the steps leading to the present ophthalmic lens packaging system includes (1) molding an ophthalmic lens in a mold comprising a posterior and anterior mold portion, (2) removing the lens from the mold and hydrating the lens, (3) introducing the aqueous packaging solution with the anionic polymer and non-ionic polyol into the container with the lens supported therein, and (4) sealing the container. Preferably, the method also includes the step of sterilizing the contents of the container. Sterilization may take place prior to, or most conveniently after, sealing of the container and may be effected by any suitable method known in the art, e.g., by autoclaving of the sealed container and its contents at temperatures of about 120 °C or higher.

The following examples are provided to enable one skilled in the art to practice the invention and are merely illustrative of the invention. The examples should not be read as limiting the scope of the invention as defined in the claims.

EXAMPLE 1

A clean hilafilcon A lens, which is a copolymer composed mainly of HEMA and NVP, was soaked in a lens packaging solution within the scope of the present invention overnight and autoclaved for 30 minutes at 121°C. The ingredients and amounts of the solution are set forth in Table 2. The carboxymethylcellulose was a medium viscosity (MV) grade (viscosity no greater than 30 cp). The XPS results for the lens are set forth below in Table 4.

TABLE 2

| Ingredients | % w/w |
|---|---|
| Sodium borate | 0.215 |
| Boric acid | 1.000 |
| Ethylenediaminetetraacetic acid (EDTA) | 0.050 |
| Carboxymethylcellulose (MV) | 1.000 |
| Glycerin | 1.000 |
| | |
| pH=7.16 | |
| Osmolality=347 mOsm/kg | |

COMPARATIVE EXAMPLE A

A clean hilafilcon A lens as used in Example 1 was soaked in a lens packaging solution outside the scope of the invention overnight and autoclaved for 30 minutes at 121 °C. The ingredients and amounts of the solution are set forth in Table 3. The XPS results for the lens are set forth below in Table 4.

TABLE 3

| Ingredients | % w/w |
|---|---|
| Sodium borate | 0.215 |

(continued)

| Ingredients | % w/w |
|---|---|
| Boric acid | 1.000 |
| EDTA | 0.050 |
| Carboxymethylcellulose (MV) | 1.000 |
| | |
| pH=7.49 | |
| Osmolality=237 mOsm/kg | |

Testing

Sample specimens prepared in Example 1 and Comparative Example A were analyzed for its atomic concentration by XPS and compared to a clean hilafilcon A lens rich in HEMA and NVP content which was not soaked in a packaging solution and autoclaved. The X-ray Photoelectron Spectrometer (XPS) utilized in this study was a Physical Electronics [PHI] Model 5600. This instrument utilized an aluminum anode operated at 300 watts, 15 kV and 27 milliamps. The excitation source was monochromatized utilizing a torodial lens system. The 7 mm filament was utilized for the polymer analysis due to the reduced sample damage and ease of photoionization neutralization. The base pressure of this instrument was $2.0 \times 10^{-10}$ torr while the pressure during operation was $1.0 \times 10^{-9}$ torr. This instrument made use of a hemispherical energy analyzer. The practical measure of sampling depth for this instrument at a sampling angle of 45° and with respect to carbon was approximately 74 angstroms. All elements were charge corrected to the $CH_x$ peak of carbon to a binding energy of 285.0 electron volts (eV).

Each of the specimens was analyzed utilizing a low resolution survey spectra [0-1100 eV] to identify the elements present on the sample surface. The high resolution spectra were performed on those elements detected from the low resolution scans. The elemental composition was determined from the high resolution spectra. The atomic composition was calculated from the areas under the photoelectron peaks after sensitizing those areas with the instrumental transmission function and atomic cross sections for the orbital of interest. Since XPS does not detect the presence of hydrogen or helium, these elements will not be included in any calculation of atomic percentages. It is also noted that atomic percentages may vary if a different instrument design, i.e., transmission function, is utilized, so that for purposes of exact reproducibility the atomic percentage numbers in the application refer to the specified instrument design, as will be understood by the skilled artisan.

The low resolution XPS survey spectra taken at a takeoff angle of 45° for the untreated lens' surfaces contained peaks for carbon, nitrogen, oxygen, boron and sodium. The analysis of the lens' material begins with the examination of the unmodified matrix (control). Table 4 below contains the XPS data for the samples. This data reflects the atomic composition over the top 74 angstroms (relative to carbon Is electrons). The percentages reflect all elements except hydrogen and helium.

TABLE 4

| | Atomic Concentration | | | | |
|---|---|---|---|---|---|
| | Carbon | Nitrogen | Oxygen | Boron | Sodium |
| Cleaned Product | 74.6 | 4.1 | 21.3 | 0.0 | 0.0 |
| Comp. Ex. A | 72.9 | 3.7 | 22.3 | 1.1 | 0.0 |
| Ex. 1 | 70.2 | 1.8 | 25.7 | 1.4 | 0.9 |

As can be seen from Table 4, the amount of nitrogen in the lens soaked in a packaging solution containing both an anionic polymer and a non-ionic polyol of the present invention is substantially reduced when compared to a lens soaked in a packaging solution containing only an anionic polymer. The substantial reduction in the amount of nitrogen present on the lens indicates that the surface of the lens has been covered with the components of the packaging solution, thereby providing a more uniform coating thereon.

It will be understood that various modifications may be made to the embodiments disclosed herein. Therefore the above description should not be construed as limiting, but merely as exemplifications of preferred embodiments. For

example, the functions described above and implemented as the best mode for operating the present invention are for illustration purposes only. Other arrangements and methods may be implemented by those skilled in the art without departing from the scope and spirit of this invention. Moreover, those skilled in the art will envision other modifications within the scope and spirit of the features and advantages appended hereto.

In view of the above, some aspects of the invention relate to:

1. A method of preparing a package comprising a storable, sterile ophthalmic device, the method comprising:

(a) immersing an ophthalmic device in an aqueous packaging solution comprising an anionic polymer and a non-ionic polyol, wherein the aqueous packaging solution has an osmolality of at least about 200 mOsm/kg and a pH in the range of about 6 to about 9;
(b) packaging the solution and the device in a manner preventing contamination of the device by microorganisms; and
(c) sterilizing the packaged solution and device.

2. The method of aspect 1, wherein the ophthalmic device is a contact lens.

3. The method of aspect 1, wherein the ophthalmic device comprises a polymerization product of a monomeric mixture comprising one or more silicone-containing monomers.

4. The method of aspect 1, wherein the anionic polymer possesses a Mark-Houwink Constant of greater than 0.6.

5. The method of aspect 1, wherein the anionic polymer possesses a Mark-Houwink Constant of greater than about 1.

6. The method of aspect 1, wherein the anionic polymer possesses a Mark-Houwink Constant of greater than about 1.6.

7. The method of aspect 1, wherein the anionic polymer in the aqueous packaging solution is a carboxy-containing polysaccharide.

8. The method of aspect 7, wherein the carboxy-containing polysaccharide is selected from the group consisting of a carboxy-containing cellulose, hyaluronate, chondroitin sulfate, guar, alginate, pectin, xanthan and mixtures thereof.

9. The method of aspect 7, wherein the carboxy-containing polysaccharide is a carboxymethylcellulose.

10. The method of aspect 1, wherein the anionic polymer in the aqueous packaging solution is a carboxy-containing vinyl polymer.

11. The method of aspect 10, where the carboxy-containing vinyl polymer is selected from the group consisting of a carbomer, polymer of acrylic acid and/or methacrylic acid and mixtures thereof.

12. The method of aspect 1, wherein the anionic polymer in the aqueous packaging solution is an anionic polypeptide.

13. The method of aspect 12, wherein the anionic polypeptide is selected from the group consisting of a poly(glutamic acid), poly(aspartic acid) and mixtures thereof.

14. The method of aspect 1, wherein the anionic polymer has a degree of substitution value of about 0.5 to about 1.5.

15. The method of aspect 1, wherein the concentration of the anionic polymer in the aqueous packaging solution is about 0.01 to about 10 % w/w.

16. The method of aspect 1, wherein the non-ionic polyol is selected from the group consisting of glycerin, ethylene glycol, poly(ethylene glycol), propylene glycol, monosaccarides, disaccharides, oligopolysaccharides or polysaccharides and mixtures thereof.

17. The method of aspect 1, wherein the anionic polymer is a carboxymethylcellulose and the non-ionic polyol is glycerin.

18. The method of aspect 1, wherein the solution further comprises a buffering agent.

19. The method of aspect 1, wherein the aqueous packaging solution coats the ophthalmic device more uniformly than that of a similar aqueous packaging solution in which the non-ionic polyol is not present in the aqueous packaging solution.

20. The method of aspect 1, further comprising hermetically sealing the ophthalmic device and the aqueous packaging solution in the package.

21. The method of aspect 20, wherein heat sterilization is performed subsequent to sealing of the package.

22. The method of aspect 1, wherein the aqueous packaging solution does not contain an effective disinfecting amount of a disinfecting agent.

23. The method of aspect 1, wherein the aqueous packaging solution does not contain a germicide compound.

24. A packaging system for the storage of an ophthalmic device comprising a sealed container containing one or more unused ophthalmic devices immersed in an aqueous packaging solution comprising an anionic polymer and a non-ionic polyol, wherein the aqueous packaging solution has an osmolality of at least about 200 mOsm/kg, a pH of about 6 to about 9 and is heat sterilized.

25. The packaging system of aspect 24, wherein the ophthalmic device is a contact lens.

26. The packaging system of aspect 24, wherein the ophthalmic device comprises a polymerization product of a monomeric mixture comprising one or more silicone-containing monomers.

27. The packaging system of aspect 24, wherein the anionic polymer possesses a Mark-Houwink Constant of greater than 0.6.

28. The packaging system of aspect 24, wherein the anionic polymer possesses a Mark-Houwink Constant of greater than about 1.

29. The packaging system of aspect 24, wherein the anionic polymer possesses a Mark-Houwink Constant of greater than about 1.6.

30. The packaging system of aspect 24, wherein the anionic polymer in the aqueous packaging solution is a carboxy-containing polysaccharide.

31. The packaging system of aspect 30, wherein the carboxy-containing polysaccharide is selected from the group consisting of a carboxy-containing cellulose, hyaluronate, chondroitin sulfate, carboxy-containing guar, alginate, pectin, xanthan and mixtures thereof.

32. The packaging system of aspect 30, wherein the carboxy-containing polysaccharide is a carboxymethylcellulose.

33. The packaging system of aspect 24, wherein the anionic polymer has a degree of substitution value of about 0.5 to about 1.5.

34. The packaging system of aspect 24, wherein the concentration of the anionic polymer in the aqueous packaging solution is about 0.01 to about 10% w/w.

35. The packaging system of aspect 24, wherein the non-ionic polyol is selected from the group consisting of glycerin, ethylene glycol, poly(ethylene glycol), propylene glycol, monosaccarides, disaccharides, oligopolysaccharides or polysaccharides and mixtures thereof.

36. The packaging system of aspect 24, wherein the anionic polymer is a carboxymethylcellulose and the non-ionic polyol is glycerin.

37. The packaging system of aspect 24, wherein the aqueous packaging solution further comprises a buffering agent.

38. The packaging system of aspect 24, wherein the aqueous packaging solution coats the ophthalmic device more uniformly than that of a similar aqueous packaging solution in which the non-ionic polyol is not present in the solution.

39. The packaging system of aspect 24, wherein package is heat sterilized subsequent to sealing of the package.

40. The packaging system of aspect 24, wherein the solution does not contain an effective disinfecting amount of a disinfecting agent.

41. The packaging system of aspect 24, wherein the aqueous packaging solution does not contain a germicide compound.

## Claims

**1.** An aqueous packaging solution having an osmolality of at least 200 mOsm/kg and a pH of 6 to 9 and comprising a non-ionic polyol and an anionic polymer, wherein the Mark-Houwink Constant of the anionic polymer is greater than 0.6.

**2.** The aqueous packaging solution according to claim 1, wherein the anionic polymer possesses a Mark-Houwink Constant of greater than 1 and preferably of greater than 1.6.

**3.** The aqueous packaging solution according to claim 1, wherein the anionic polymer in the aqueous packaging solution is a carboxy-containing polysaccharide, preferably selected from the group consisting of a carboxy-containing cellulose, hyaluronate, chondroitin sulfate, guar, alginate, pectin, xanthan and mixtures thereof and most preferably is a carboxymethylcellulose.

**4.** The aqueous packaging solution according to claim 1, wherein the anionic polymer in the aqueous packaging solution is a carboxy-containing vinyl polymer, preferably selected from the group consisting of a carbomer, polymer of acrylic acid and/or methacrylic acid and mixtures thereof.

**5.** The aqueous packaging solution according to claim 1, wherein the anionic polymer in the aqueous packaging solution is an anionic polypeptide, preferably selected from the group consisting of a poly(glutamic acid), poly(aspartic acid) and mixtures thereof.

**6.** The aqueous packaging solution according to claim 1, wherein the anionic polymer has a degree of substitution value of 0.5 to 1.5.

**7.** The aqueous packaging solution according to claim 1, wherein the concentration of the anionic polymer in the aqueous packaging solution is 0.01 to 10 % w/w.

**8.** The aqueous packaging solution according to claim 1, wherein the non-ionic polyol is selected from the group consisting of glycerin, ethylene glycol, poly(ethylene glycol), propylene glycol, monosaccharides, disaccharides, oligopolysaccharides or polysaccharides and mixtures thereof.

**9.** The aqueous packaging solution according to claim 1, wherein the anionic polymer is a carboxymethylcellulose and the non-ionic polyol is glycerin.

**10.** Use of an aqueous packaging solution according to the claims 1 to 9 for packaging and storing a sterile ophthalmic device.

**11.** Use of an aqueous packaging solution according to claim 10, wherein the ophthalmic device is a contact lens.

**12.** Use of an aqueous packaging solution according to claim 10, wherein the ophthalmic device comprises a polymerization product of a monomeric mixture comprising one or more silicone-containing monomers.

**13.** Use of an aqueous packaging solution according to claim 10, wherein the aqueous packaging solution coats the ophthalmic device more uniformly than that of a similar aqueous packaging solution in which the non-ionic polyol is not present in the aqueous packaging solution.

**14.** Method for preparing an ophthalmic lens packaging system, the method comprising:

(a) molding an ophthalmic lens in a mold comprising a posterior and anterior mold portion
(b) removing the lens from the mold and hydrating the lens
(c) introducing an aqueous packaging solution according to the claims 1 to 9 and
(d) sealing the container.

**15.** The method according to claim 14, wherein heat sterilization is performed subsequent to sealing of the package.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5882687 A
- US 4136250 A
- US 4153641 A
- US 4740533 A
- US 5034461 A
- US 5070215 A
- US 5260000 A
- US 5310779 A
- US 5358995 A
- WO 9631792 A
- US 5449729 A
- US 5512205 A
- US 4910277 A
- US 4954587 A
- US 5010141 A
- US 5079319 A
- US 5321108 A
- US 5387662 A
- US 3408429 A
- US 3660545 A
- US 4113224 A
- US 4197266 A
- US 4555732 A

### Non-patent literature cited in the description

- **L. H. SPERLING.** Introduction to Physical Polymer Science. Wiley-Interscience, A John Wiley & Sons, Inc, 2001